# EUROPEAN PATENT SPECIFICATION

(11) **EP 1 706 168 B1**
(45) Date of publication and mention of the grant of the patent: **06.11.2013**
(21) Application number: 04810795.7
(22) Date of filing: 12.11.2004
(51) Int. Cl.: A61F 2/01

(54) **MEDICAL DEVICE ANCHOR AND DELIVERY SYSTEM**
MEDIZINPRODUKTEVERANKERUNGS- UND ABGABESYSTEM
ANCRAGE POUR DISPOSITIF MEDICAL ET SYSTEME DE DISTRIBUTION

(30) Priority: 12.11.2003 US 705226; 04.11.2004 US 980828
(43) Date of publication of application: 04.10.2006
(73) Proprietor: Nitinol Devices and Components, Inc., Fremont, CA 94539 (US)
(72) Inventor: RAVENSCROFT, Adrian, Carver, MA 02330 (US); KLESHINSKI, Stephen J., Carver, MA 02330 (US)
(74) Representative: Papula Oy
(86) International application number: PCT/US2004/037738
(87) International publication number: WO 2005/046783

(56) References cited:
- WO-A1-97/13463
- US-A- 6 007 558
- US-B1- 6 231 589
- US-B1- 6 231 589

## Description

### TECHNICAL FIELD

Recent advances in medical technology have resulted in the development of a variety of medical devices for permanent or temporary implantation in the human body. Effective positioning of such devices can prove to be a very difficult task, and maintaining an implanted device in a desired position for an extended period of time is often more difficult. This is particularly true if the implanted device is to remain only temporarily and is designed to facilitate subsequent removal.

### BACKGROUND OF THE INVENTION

A number of medical implant devices are designed to collapse for insertion within a catheter or other delivery unit and to expand to a predetermined shape when ejected after delivery. Many of these self expanding devices rely primarily upon the contact between the device and the wall of a body vessel or passageway to maintain the device in position after the delivery unit is removed. Unfortunately, changes in the dimensions of the body vessel or passageway or variations in the flow of blood or other fluids there through can cause the medical implant to migrate and change position.

It is extremely important that a medical implant device be properly positioned and oriented, and that this position and orientation be maintained. Otherwise, effective performance of such therapeutic devices will not be achieved. It is often very difficult to move such a device into position with the desired orientation, and once this is achieved, it is critical that no further motion occur.

In an attempt to prevent migration of a medical implant device, rigid hooks are often formed on the device to engage the wall of a body vessel or passageway as the implant device expands into contact with the wall. After a few weeks, the endothelium layer grows over rigid hooks which will not easily bend under the influence of withdrawal pressure, and the medical implant device will be locked in place by the embedded hooks. This may be acceptable for a permanent implant, but rigid hooks are not a viable option if the medical implant device is to be removed after several weeks or months.

To facilitate removal of a previously implanted medical device by withdrawal of the anchoring hooks from an enveloping endothelium layer without risking substantial damage to the wall of a body vessel or passageway, the hooks have been formed to straighten when subjected to a withdrawal force greater than a maximum migration force. U.S. Patent Nos. 6,007,558 and 6,258,026 to Ravenscroft, et al show hooks which are formed to bend and straighten in response to a withdrawal force, while U.S. Patent Nos. 4,425,908 to Simon, 4,817,600 to Herms, et al, 5,108,418 to Lefebvre, 5,133,733 to Rasmussen, et al, 5,242,462 to E1-Nounou, et al, 5,370,657 to Irie, 5,601,595 to Smith, 5,800,457 to Gelbfish, and 5,853,420 to Chevillon, et al all disclose expandable medical implant devices; many with anchoring hooks.

Anchoring hooks, although effective in many instances, are subject to a number of disadvantages which can make it difficult to properly position and maintain the position of a medical implant device. In prior devices, the anchoring hooks are engaged due to the expansion of the device into contact with the wall of a body vessel or passageway, and if the device moves from a desired position during expansion and contact with the wall occurs, the device cannot be easily repositioned. The anchoring function of the hooks is not separable from the expansion of the device.

In cases where the operation of the hooks is tied to the expansion of a medical implant device, there can be instances where one or more of the hooks fails to properly engage the wall of a body vessel or passageway causing the device to become off center. Sometimes movement of the device longitudinally will engage the errant hooks, but this movement can also alter the position of the device.

Also, the configuration of a hook which curves in a single direction from a shaft to a pointed end can prove to be a disadvantage. When hooks are used to anchor a medical implant device within a blood vessel, it is important that the hook be oriented to curve in the direction of normal blood flow through the vessel as it engages the vessel wall. Thus when engaged, the hook will extend from the shaft toward the point substantially in the direction of the longitudinal axis of the blood vessel, and will effectively resist migration of the medical implant device in response to pressure thereon from blood flow in the normal direction through the blood vessel. However, there are conditions which can result in a backflow of blood in a blood vessel, and pressure on the device and the anchoring hooks resulting from such backflow can cause the hooks to back out and disengage from the vessel, thus changing the orientation of the device within the blood vessel and causing deleterious changes in the performance of the implant.

Finally, even if the hooks of an implant device are properly engaged with a vessel wall, there are conditions which result in the subsequent outward expansion of the vessel wall to an extent where the hooks tend to become disengaged.

### SUMMARY OF THE INVENTION

An exemplary method is presented for positioning and anchoring a medical implant device which includes positively propelling one or more anchors through a body wall subsequent to a medical implant device connected to the anchor reaching a desired position and coming to rest.

An object of the present invention is to provide a novel and improved medical device anchor and delivery system wherein one or more anchors are positively propelled through a body wall. Once an anchor has passed through the wall, it expands outwardly from at least two opposed sides of an anchor shaft.

In an example of the medical device anchor is designed to penetrate a body wall from a first side to a second side and to expand outwardly from at least two opposed sides of an anchor shaft after penetration.

A example of the medical device anchor is designed to penetrate the wall of a body vessel from a first side to a second side and to expand outwardly from an anchor shaft in a unique manner after penetration. The expanded anchor is designed to be loaded in compression against the second wall of the vessel and to change in configuration to increase the anchoring function provided thereby in response to forces applied thereto at an angle to the longitudinal axis of the vessel.

Yet an object of the present invention is to provide a novel and improved medical device anchor designed to penetrate the wall of a body vessel from a first side to a second side and to expand outwardly from an anchor shaft in a unique manner after penetration. The anchor expands outwardly from the anchor shaft into one or more loops with each loop curving back to cross the anchor shaft. The section of the loop which crosses the anchor shaft is formed to engage the second wall of the vessel and to load the anchor in compression against the second wall of the vessel in response to forces which are applied to a medical device attached to the anchor or which result from expansion of the vessel wall.

In a further example, the medical device anchor and delivery system is provided wherein one or more anchors are positively propelled through a body wall subsequent to a medical implant device connected to the anchors reaching a desired position and coming to rest. The anchor delivery system facilitates removal and reinsertion of the anchors without requiring that the medical implant device connected thereto be compressed and/or removed.

In a further example, the anchor and anchor delivery system for a medical implant device is provided to anchor the device in position within a blood vessel or other body passageway. Once the medical implant device has been positioned and expanded into contact with the wall of the blood vessel or body passageway, the anchor delivery system then positively propels one or more anchors through the vessel or passageway wall where the anchors expand outwardly on opposite sides of an anchor shaft. The anchor delivery system permits the anchors to be withdrawn and then reinserted through the wall without the necessity to collapse the medical implant device.

In a further example, the anchor and anchor delivery system for a medical implant device is provided to anchor the device in position within a blood vessel or other body passageway while facilitating the subsequent withdrawal of the device. The anchor delivery system positively propels one or more anchors through the wall of a blood vessel or body passageway once the medical implant device has expanded into contact with the wall, and the anchors then expand outwardly from opposite sides of an anchor shaft. The anchors are formed to contract back toward the longitudinal axis of the anchor shaft in response to a predetermined force to permit withdrawal through the wall.

In a further example, a anchor and anchor delivery system is provided for a blood clot filter where the delivery system includes elongate, tubular filter legs which house the anchors. Once the filter legs are ejected from a catheter or delivery tube and expand into contact with the blood vessel wall, the anchor delivery system positively propels the anchors outwardly from the filter legs and through the blood vessel wall from a first side to a second side where the anchors expand outwardly from an anchor shaft against the second side of the wall. Each anchor is formed to contract back toward the longitudinal axis of its anchor shaft in response to a predetermined force to permit withdrawal through the wall, and this permits the anchors to be withdrawn back into the filter legs and then again propelled through the blood vessel wall without collapsing the filter legs.

In a further example, a anchor delivery system for a blood clot filter is provided where the delivery system includes elongate, tubular filter legs which house the anchors and which expand into contact with a blood vessel wall. A side opening is formed in the portion of the filter leg which will contact the blood vessel wall, and the filter leg is designed to facilitate ejection of the anchor through the side opening transverse to the filter leg. Once the filter legs expand into contact with the blood vessel wall, the anchor delivery system positively propels the anchors laterally outward from the side openings in the filter legs and through the blood vessel wall from a first side to a second side where the anchors expand outwardly from an anchor shaft against the second side of the blood vessel wall.

These and other objects of the present invention are achieved by the invention as further specified in the appended claims.

### BRIEF DESCRIPTION OF THE DRAWINGS

Figure 1 is a sectional view showing a blood clot filter with anchors formed in accordance with the present invention mounted within a catheter;

Figure 2 is a perspective view showing the anchor support hub and leg retention sleeve of Figure 1;

Figure 3 is a perspective view showing the locking sleeve for the leg retention sleeve of Figure 2;

Figure 4 is a sectional view showing the operating mechanism for the locking sleeve and anchor support hub of Figure 1;

Figure 5 is a perspective view showing a spring powered triggering unit at the proximal end of the catheter of Figure 1 for propelling the anchor support hub;

Figure 6 is a perspective view of the deployed blood clot filter of Figure 1;

Figure 7 is a perspective view of a deployed anchor for the blood clot filter of Figure 6;

Figure 8 is a perspective view of a second embodiment of a deployed anchor of the present invention;

Figure 9 is a perspective view of a third embodiment of a deployed anchor of the present invention;

Figure 10 is a sectional view of a single anchor and anchor delivery system of the present invention;

Figure 11 is a perspective view of a fourth embodiment of a deployed anchor of the present invention which deploys to form a closed loop having a wall engaging section which crosses over and extends beyond the anchor shaft;

Figure 12 is a perspective view of a fifth embodiment of a deployed anchor of the present invention which deploys to form a closed loop having a wall engaging section which crosses under and extends beyond the anchor shaft;

Figure 13 is a view in side elevation of an anchor guide boot which is secured to the end of an anchor containing blood clot filter leg,

Figure 14 is a sectional view of the anchor guide boot of Figure 13,

Figure 15 is a sectional view of a modification of the anchor guide boot of Figure 14.

Figure 16 is a perspective view of a deployed blood clot modified to eject anchors from the side of the filter legs above the distal ends of the legs with the anchors deployed,

Figure 17 is a view in front elevation of an end section of a filter leg of the filter of Figure 16 with an anchor partially deployed, and

Figure 18 is a view in front elevation of an end section of a filter leg of the filter of Figure 16 with an anchor fully deployed.

### BEST MODE FOR CARRYING OUT THE INVENTION

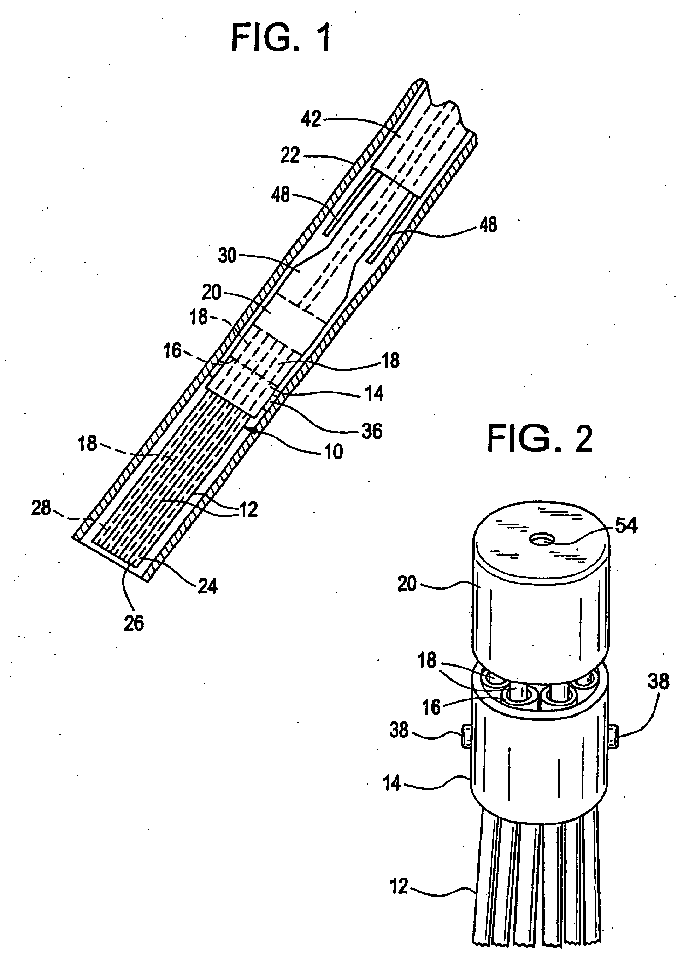

Referring to Figures 1-2, a blood clot filter which includes anchors in accordance with the present invention is illustrated generally at 10. This filter, shown for illustration as a vena cava filter, is formed with a plurality of elongate legs 12 which are secured to, and extend outwardly from a leg retention sleeve 14. The elongate legs are formed by small, open ended tubes each having a first open end 16 which opens at the leg retention sleeve. A plurality of long anchor shafts 18 are attached at a distal end to an anchor support hub 20 which is spaced from the leg retention sleeve when the vena cava filter is collapsed within a catheter or delivery tube 22. Each shaft 18 extends from the anchor support hub 20 into the first open end 16 of a tubular leg 12 and through the leg to a distal end 24 at a point adjacent to a second open end 26 of the tubular leg. An anchor 28 is formed at the distal end of each shaft 18 in a manner to be described.

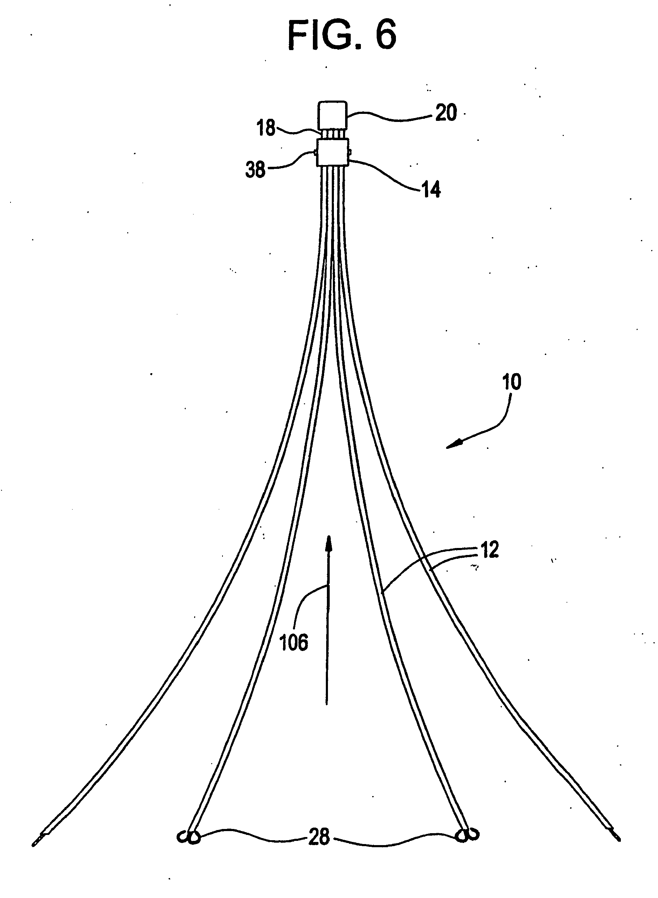

The elongate legs 12 and the long anchor shafts 18 are formed of a material which will permit them to be compressed toward the longitudinal axis of the filter 10 for delivery by a catheter 22. Once the filter is ejected from the catheter, the legs 12 and the shafts 18 are designed to expand outwardly from the filter longitudinal axis as shown in Figure 6 to bring the legs into contact with the wall of a blood vessel. Although spring metal and suitable plastics can be used to form the legs 12 and/or the shafts 18, it is preferable to form the anchor shafts 18 and in most cases the legs 12 of a suitable shape memory material. If a temperature responsive shape memory material such as nitinol is used, transition between the martensitic and austenitic states of the material can be achieved by temperature transitions relative to a transition temperature. In the martensitic state, the material softens, thereby permitting a filter formed thereof to be compressed and loaded into a catheter. If the transition temperature of the material is set at, or near to normal body temperature, then the filter legs will pass to the austenitic state when the filter is ejected from the catheter and expand to regain a memorized shape.

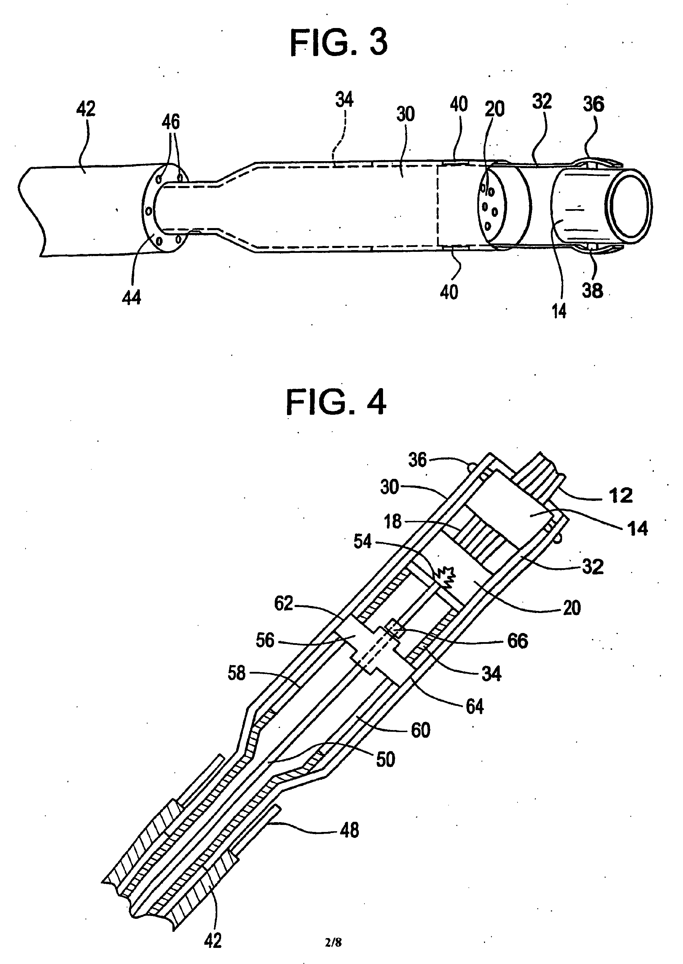

For delivery through the catheter 22, the leg retention sleeve 14 is locked to the anchor support hub 20 by a locking sleeve 30 which surrounds both the anchor support hub and the leg retention sleeve when in the locking position as shown in Figure 1. In the unlocked position, the locking sleeve is moved longitudinally back away from the leg retention sleeve as shown in Figure 3. Two spring arms 32 are connected at one end to a housing 34 behind the anchor support hub and extend outwardly over opposite sides of the leg retention sleeve. The free end of each of the spring arms is curved to form an arcuate latch member 36 which overlies and, in the locking position of Figure 1, engages a locking projection 38 formed on the leg retention sleeve. When the locking sleeve 30 moves toward the locking position over the leg retention sleeve 14, it forces the spring arms 32 and 34 together and the arcuate latch members engage the locking projections. As the locking sleeve reaches the full locking position of Figure 1, the arcuate latch members slide into slots 40 in the locking sleeve and the leg retention sleeve is positively locked to the anchor support hub. However, as the locking sleeve is moved longitudinally away from the leg retention sleeve, the arcuate configuration of the latch members 36 permits them to slip out of the slots 40, and as the locking sleeve moves further, the spring arms 32 move outwardly causing the arcuate latch members to disengage the locking projections 38.

The locking sleeve 30 is mounted for movement toward and away from a centering shaft 42 which extends from a distal end 44 adjacent to the vena cava filter 10 back to the entry end of the catheter 22. The distal end of the centering shaft is formed with a plurality of spaced lumens 46, each of which mounts one of a plurality of centering arms 48. The centering shaft moves these centering arms out of the catheter 22 behind the vena cava filter, and these centering arms then expand outwardly to engage the vessel wall and center the leading end of the filter. These centering arms can be formed of spring metal or plastic, but are preferably formed of shape memory material such as nitinol.

To control the positioning of the vena cava filter 10 and subsequent ejection of the anchors 28 from the second open ends of the legs 12, an elongate drive shaft 50 extends from the entry or proximal end 52 of the catheter 22 through the catheter to a releasable connection 54 with the anchor support hub 20. This releasable connection can be any suitable connection which facilitates release of the drive shaft from the anchor support hub by manipulation of the drive shaft at the proximal end of the catheter such as a threaded connector as shown, a hook and eye connector, engaging hook connectors, and known twist engagement and release connectors. This drive shaft passes through the centering shaft 42 and is both rotationally and longitudinally movable relative thereto.

As shown in Figure 4, the drive shaft passes through and is both rotationally and longitudinally movable relative to a locking sleeve operator 56 which passes through slots 58 and 60 in the housing 34. The locking sleeve operator is secured at 62 and 64 to the locking sleeve 30 and operates to move the locking sleeve away from the leg retention sleeve 14 as the locking sleeve operator moves away from the leg retention sleeve in the slots 58 and 60. The drive shaft operates to move the locking sleeve from the locked position by means of a stop 66 secured to the drive shaft and positioned to engage the locking sleeve operator.

When the catheter 22 reaches a desired position within a blood vessel, the vena cava filter 10 and centering arms 48 are exposed by either ejecting them from the catheter or drawing the catheter back from around them. Now the elongate legs 12 and centering arms 48 will expand outwardly into engagement with the vessel wall. However, the anchors 28 will remain enclosed within the elongate legs, and this permits the vena cava filter to be moved relative to the blood vessel after expansion of the elongate legs until an exact position is attained. If a substantial position change is required, the centering arms and vena cava filter can be drawn back into the catheter and subsequently redeployed in a new position.

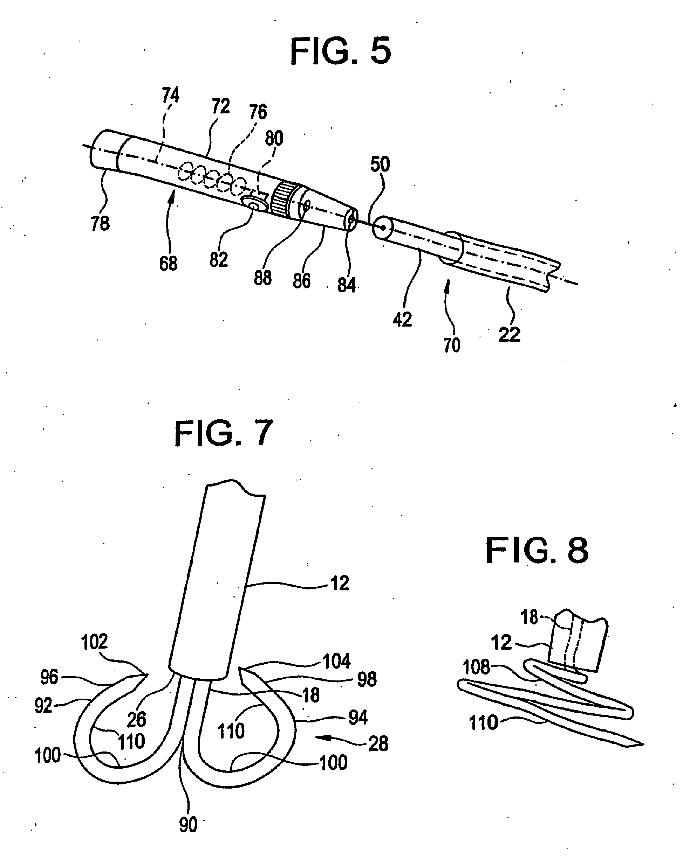

With the vena cava filter in the desired position within a blood vessel and the elongate legs 12 engaging the vessel wall, the anchors 28 are now positively ejected out from the second open ends 26 of the elongate legs so as to penetrate through the vessel wall. To achieve this positive ejection of the anchors subsequent to engagement of the elongate legs with the vessel wall with sufficient force to result in penetration of the vessel wall, the drive shaft 50 is connected to a triggering unit 68 at the proximal or entry end 70 of the catheter 22. This triggering unit can be formed by a number of known units capable of imparting a longitudinal force to the drive shaft. An electrically powered solenoid unit can be used for this purpose as well as a number of spring powered units. In Figure 5, the triggering unit is formed by a conventional ballistic-type lancer of the type commonly used to cause a needle to puncture a patient's skin to provide a blood sample. Such lancers include a hollow body 72 which contains a plunger 74 capable of moving axially back and forth within the body. The plunger is surrounded by a coil spring 76 which becomes compressed when the plunger is pulled back and armed by an end knob 78. The armed plunger is held in place by a trigger 80 which is activated to release the plunger by a button 82. When the plunger is released, the coil spring 76 propels the plunger toward an opening 84 in a nose cap 86 attached to the hollow body. For normal use of the ballistic type lancer, a needle is secured to the end 88 of the plunger and is propelled by the released plunger out through the opening 84 and into the skin of a patient. In Figure 5, the drive shaft 50 is secured to the end 88 of the plunger, and when the armed plunger is released, the drive shaft is propelled longitudinally to drive the anchor support hub 20 toward the leg retention sleeve 14. This causes the long shafts 18 to move longitudinally through the elongate legs 12 to propel the anchors out and through the vessel wall. Figure 6 illustrates an expanded vena cava filter 10 with the anchors 28 in the configuration that they would assume after passing through the vessel wall. The structure and operation of these anchors will be subsequently described.

A significant advantage of the vena cava filter 10 is that it can be repositioned even after the anchors are in place without the necessity to withdraw the complete filter back into the catheter 22. So long as the elongate legs are in contact with the vessel wall, the anchors 28 can be withdrawn from the vessel wall and back into the elongate legs by causing the drive shaft 50 to move the anchor support hub 20 away from the leg retention sleeve 14. Now the vena cava filter can be repositioned, the plunger 74 of the triggering unit 68 can be rearmed, and the anchors can again be ejected to pierce the vessel wall.

Once the vena cava filter 10 is properly positioned and anchored within a blood vessel, the drive shaft 50 is disconnected from the anchor support hub 20 and is pulled away from the anchor support hub causing the stop 66 to engage and move the locking sleeve operator 56 away from the anchor support hub. This results in movement of the locking sleeve 30 away from the leg retention sleeve 14 so that the spring arms 32 spring outwardly and the latch members 36 disengage from the locking projections 38. Now the centering shaft 42, locking sleeve 30, drive shaft 50 and housing 34 may be drawn back through the catheter 22 leaving the vena cava filter in place within the blood vessel.

To subsequently remove a previously anchored vena cava filter, standard body retrieval devices which engage the filter body may be used. For example, a hook to be engaged by a retrieval device can be attached to the anchor support hub 20.

The anchors 28 are formed at the proximal ends of the long anchor shafts 18, and within the elongate legs 12 the anchors assume the same configuration as the shafts with which they are integrally formed. The shafts conform in configuration to the internal configuration of the elongate legs so as to easily move longitudinally within the elongate legs, and usually the shafts will be cylindrical with a pointed end which forms the leading end of the anchor. An enlarged view of the anchor of Figure 6 is shown in Figure 7.

Referring to Figure 7, the tubular anchor shaft 18 is split down the center at 90 to form the opposed arms 92 and 94 of the anchor. The inner surfaces 96 and 98 of each of the arms is flat while the remaining surface 100 of each arm is arcuate, so that when the inner surfaces of the arms are contacting, a straight tubular end section is formed on the end of each long shaft 18. The pointed end of each long shaft forms the pointed ends 102 and 104 on the arms 92 and 94 of the anchor.

The expanded shape memory configuration of the anchors 28 is shown in Figures 6 and 7. Each anchor with the inner surfaces 96 and 98 in contact is ejected from an elongate leg 12 in a straight configuration when the anchor support hub 20 is driven toward the leg retention sleeve 14. The pointed lead end of each anchor will pierce the wall of a blood vessel so that the entire anchor passes through the vessel wall, at which point the anchor expands to its shape memory configuration shown in Figure 7. Now the end 26 of the elongate leg engages the inner surface of the blood vessel wall while the pointed ends 102 and 104 of the arms 92 and 94 engage the outer surface of the blood vessel wall. It is important to note that portions of the expanded anchor, in this case the arms 92 and 94, extend outwardly on opposite sides of the shaft 18 so that forces in either direction in the plane of the anchor arms will not dislodge the anchor in the manner which can occur with a single hook which extends outwardly in only one direction from a support shaft. To provide additional protection from accidental dislodgement, the anchors 28 are oriented as shown in Figure 7 so that the opposed arms 92 and 94 of the anchor expand transversely to the longitudinal direction 106 of blood flow through the filter 10. Thus the forces created by direct or reverse blood flow cannot dislodge the anchor, but since the anchor arms are each formed from half of a shaft 18 of a very small diameter, a withdrawal force along the longitudinal axis of the shaft will permit the anchor arms to come together to facilitate anchor withdrawal from the vessel wall.

It is important to note that the anchor arms 92 and 94 curve outwardly and back toward the shaft 18 to engage the outside surface of the vessel wall. This causes the anchor to be loaded in compression against the vessel wall when forces normal to the longitudinal axis of the vessel are applied to a medical device attached to the anchor. This compression aspect greatly enhances the anchoring function provided by the anchor and facilitates the effective use of very small, fine anchor components.

The anchors 28 may take a number of forms so long as the anchor expands from a straight configuration from within an elongate leg 12 to a shape memory configuration where the anchor extends outwardly on at least two opposite sides of the shaft 18. In Figure 8, the anchor 28 expands to a spiral configuration so as to extend completely around the shaft 18. Here the shaft is not split as shown in Figure 7, but instead the intact end of the shaft is used to form the spiral 108. In all cases, first end of the anchor to emerge from an elongate leg 12 is a straight section 110 bearing the anchor point, and this section passes through a blood vessel wall before following sections which will form curves emerge. Both the anchors of Figures 7 and 8 tend to flatten by spring action against the vessel wall after expanding.

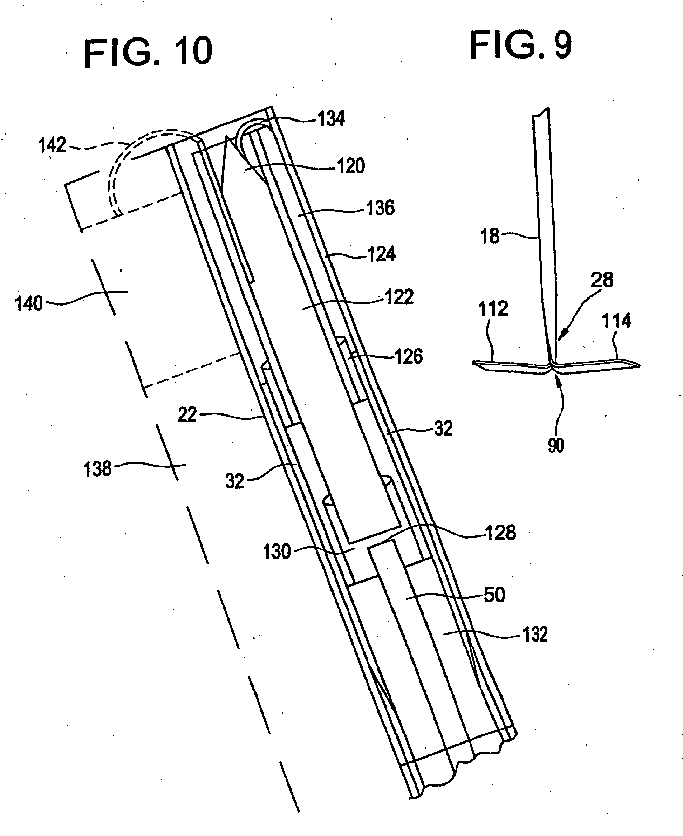

To form the anchor 28 of Figure 9, the shaft 18 is flattened at the end and split at 90 to form two opposed , flat arms 112 and 114 which expand outwardly on opposite sides of the shaft. These arms emerge from the elongate leg 12 as a straight section which passes through the vessel wall and then splits and bends outwardly at 116 and 118 to form the arms. These arms lie against the outer surface of the vessel wall and in a vena cava filter, are oriented transverse to the longitudinal direction of blood flow through the filter.

For some medical applications, a need has arisen for a single anchor to tether a device within a body vessel or to a body wall. An apparatus similar to that previously described with reference to the multiple anchor vena cava filter 10 can be employed to deploy the single anchor 120 of Figure 10. The single anchor 120 is formed at the distal end of an anchor shaft 122 mounted in an elongate tube 124, Both the shaft 122 and the tube 124 are formed of shape memory material as described relative to the elongate legs 12 and long shafts 18, but are normally much shorter in length than the elongate legs and shafts 18. A tube retention sleeve 126 retains the single tube 124 in the same manner that the leg retention sleeve 14 operates to retain the elongate legs 12, and this tube retention sleeve is engaged by a locking sleeve (not shown) and spring arms 32 operative in the manner previously described. A drive shaft 50 is connected at the entry end of the catheter 22 to a triggering unit 68, and is also connected to a releasable connection 128 similar to the releasable connection 54. This releasable connection is firmed in a shaft support hub 130 normally spaced from the tube retention sleeve 126 which is connected to the proximal end of the anchor shaft.

The drive shaft 50 is movable in a control shaft 132 similar to the centering shaft 42 which operates to move the shaft support hub and tube retention sleeve longitudinally to expel the tube 124 containing the anchor 120 from the catheter 22. The tube 124 will now assume a predetermined shape to position the anchor relative to a body wall which will receive the anchor. Now the triggering unit 68 can be operated to cause the drive shaft 50 to move the shaft support hub 130 toward the tube retention sleeve 126 to drive the anchor 120 through the body wall. The anchor 120 is formed of shape memory material and can take the form and operate in the manner of any of the anchors previously described. Once the anchor is delivered, the spring arms 32 can be operated to release the tube retention sleeve 126, and the drive shaft can be released from the releasable connection 128 so that the drive and control shafts, and in some cases the catheter, can be withdrawn. If the purpose of the anchor is to anchor the catheter in position, then a tether 134 is provided between the catheter and the anchor, and the catheter will not be withdrawn with the drive and control shafts.

In some instances, the catheter 22 may be a dual lumen catheter having a first lumen 136 containing the described anchor mechanism and a second lumen 138 containing an in implantable medical device 140 to be anchored by the anchor 120. In this case, a tether 142 is connected between the anchor and the implant able medical device, and once the anchor is in place, the implantable medical device is ejected from the catheter.

When it is possible to use the catheter to properly position the anchor 120 relative to a body wall, the tube 124 and tube retention sleeve 126 can be eliminated and replaced by the catheter lumen. Now the drive shaft 50 will drive the shaft support hub 130 longitudinally to drive the anchor from the catheter lumen and through the body wall.

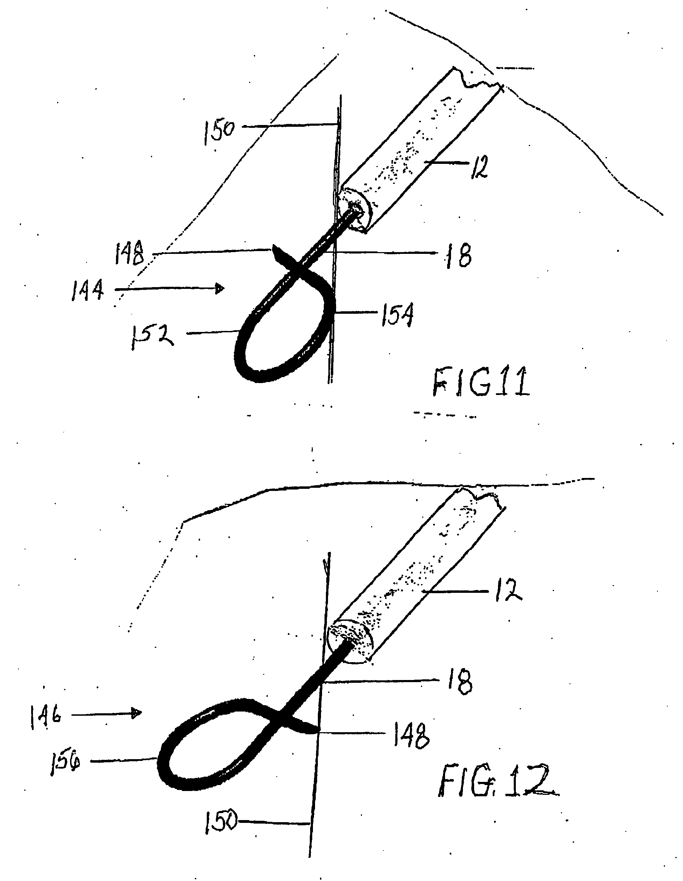

Figures 11 and 12 show anchors 144 and 146 respectively which each form a single, closed loop in the expanded shape memory configuration. Each of the anchors 144 or 146 is ejected from an elongate leg 12 in a straight configuration coextensive with the long anchor shaft 18 when the anchor support hub 20 is driven toward the leg retention sleeve 14. The end of each anchor, which may be pointed as indicated at 148, will pierce the wall 150 of the vessel containing the vena cava filter 10 or other medical implant device to be anchored, so that the entire anchor passes through and expands against the outer surface of the vessel. In its shape memory expanded configuration, the anchor 144 extends arcuately outwardly from the anchor shaft and loops back to cross over and extend beyond the anchor shaft to form a single closed loop 152. The loop 152 engages the outer surface of the vessel wall 150 at 154 and is loaded in compression against the vessel wall; a compression which increases in response to forces applied in any direction which tend to force the loop 152 further against the vessel wall. As these forces increase, the loop 152 changes configuration and decreases in size becoming more rigid as a greater portion of the loop is forced across the anchor shaft 18, thereby increasing the anchoring force of the anchor.

Unlike the anchor 144 which is oriented to be confined in the angular space between the anchor shaft 18 and the vessel wall 150, the anchor 146 is oriented to be outside this angular space. This anchor in its shape memory expanded configuration extends arcuately outwardly from the anchor shaft and loops back to cross under and extend beyond the anchor shaft to form a single closed loop 156 which is loaded in compression against the vessel wall. However, due to the orientation and configuration of the anchor 146, as forces on the anchor increase, the loop straightens rather than decreasing in size and may be withdrawn with less force than that required to withdraw the anchor 144.

Both the anchors 144 and 146 can be configured to provide a double looped anchor by splitting the shaft 18 and forming double, opposed closed loops similar to the open loops formed by the arms 92 and 94 of figure 7. However both the double closed loops of the modified anchors 144 and 146 would extend arcuately back over or under the anchor shaft in the manner shown by figures 11 or 12.

It may be desirable to insure that the distal end 24 of an anchor containing filter leg 12 cannot follow an ejected anchor through the sidewall of a blood vessel once the anchor is deployed. This can be accomplished in accordance with this invention by forming a side opening in the portion of the filter leg which will contact the vessel wall with this side opening being spaced above the distal end of the filter leg. The anchor is then ejected through this side opening laterally of the filter leg once the filter leg has expanded into contact with the vessel wall. The anchor will now pass through the vessel wall at a point above the distal end of the filter leg thereby positively precluding the distal end of the filter leg from following the anchor through the vessel wall.

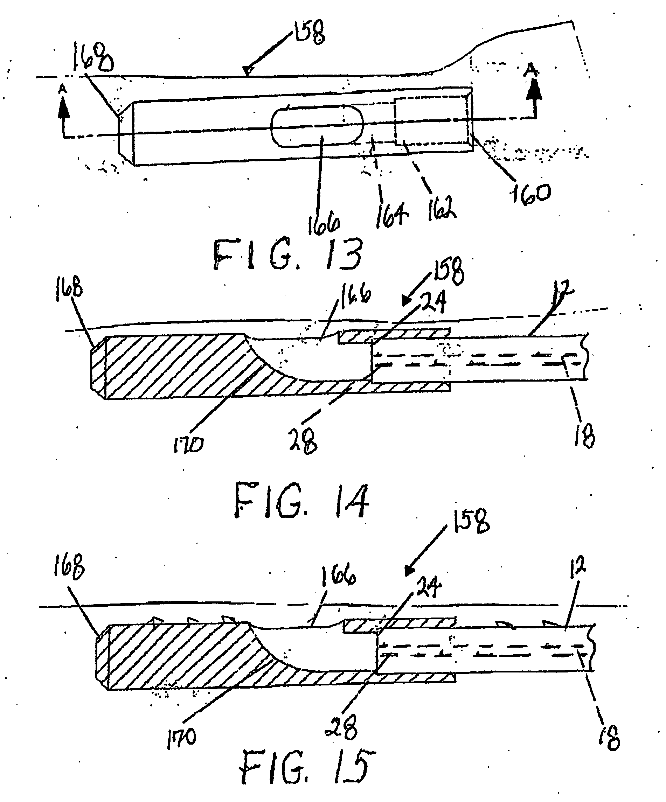

It has been found to be advantageous to attach a separate anchor guiding boot 158 of the type shown in Figures 13, 14 and 15 to the distal end 24 of each anchor containing filter leg. The anchor guiding boot has an open end 160 which opens into an internal seat 162 for the distal end of the filter leg. The end of the filter leg may be secured within the seat 162 by any known means such as by a friction fit, welding, heat expansion or bonding. An internal passage 164 connects the seat 162 to a side opening 166 formed in the anchor guiding boot, and this side opening is spaced from the closed end 168 of the anchor guiding boot. The internal passage is closed by a curved, guidewall 170 which curves upwardly from the lower end of the opening 166 to the opposite side of the internal passage.

When the triggering unit 68 is activated, each of the long anchor shafts 18 move an anchor 28 toward the closed end 168 of an anchor guiding boot 158 and into engagement with the curved, guidewall 170 which closes the internal passage 164. The anchor is then guided along the curved, guidewall. causing the shaft 18 to bend as the anchor is ejected out through the side opening 166 and laterally through the wall of the blood vessel. The anchor guiding boot 158 may be formed of tantalum to provide high feasibility under fluoroscopy.

To prevent longitudinal movement of a filter leg 12 relative to the blood vessel caused by the force applied to the curved, guidewall 170 by the ejecting anchor 28, barbs 172 may be formed on either the anchor guiding boot 158, the filter leg 12 or both. These barbs engage the blood vessel wall when the filter leg contacts the vessel wall, and are inclined to penetrate and prevent longitudinal movement of the filter leg toward the closed end 168 of the anchor guiding boot.

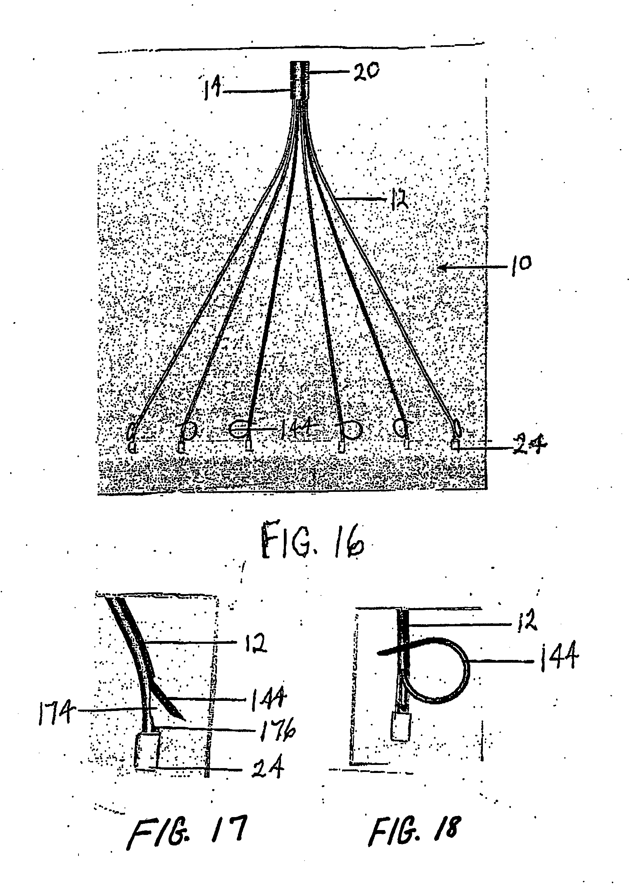

To eliminate the need for the anchor guiding boot 158, a side opening 174 to facilitate lateral anchor ejection when the triggering unit 68 is activated can be formed directly in a filter leg 12 and spaced above the distal end 24 thereof as shown in Figures 16-18. The tubular filter leg is closed between the lower end of the side opening 174 and the distal end of the filter leg so that the anchor will be ejected laterally of the filter leg through the side opening. This closure may be formed by a curved wall 176 which curves upwardly from the lower end of the side opening across the tubular interior of the filter leg. The filter 10 of Figure 16 is shown in the expanded configuration with the anchors 144 deployed laterally through the side openings 174. Figure 17 shows this anchor partially deployed, while Figure 18 shows this anchor fully deployed.

## Claims

1. A medical device anchor and delivery system for propelling an anchor through a body wall from a first side to a second side where said anchor expands against said second side, comprising:
an anchor shaft (18) having first and second opposed ends,
an expandable anchor (28) at the second end of said anchor shaft (18) having one or more anchor sections, said expandable anchor (28) having a first collapsed configuration wherein said anchor (28) is substantially coextensive with said anchor shaft (18) and a second expanded configuration, and
an elongate tube having an open proximal end and a distal end with an exit opening for said anchor (28) being formed in said tube at or adjacent to but spaced from said distal end, said tube containing said anchor shaft (18) with said expandable anchor (28) in said collapsed configuration adjacent to said exit opening,
***characterized in that***
said medical device anchor further comprises a drive shaft (50) connected to a triggering unit (68) mounted to engage said first end of said anchor shaft (18) and move said anchor shaft (18) longitudinally toward the second end of said elongate tube to propel said expandable anchor (28) outwardly from said exit opening so that the entire expandable anchor (28) passes through and expands against the outer surface of the vessel, and that
in the second expanded configuration of said expandable anchor (28), said one or more anchor sections extend outwardly from said anchor shaft (18) into one or more loops with each loop curving back to cross the anchor shaft (18).

2. The medical device anchor and delivery system of claim 1 wherein the expandable anchor at the second end of said anchor shaft (18) is formed integrally with the anchor shaft (18) by splitting the anchor shaft (18) longitudinally at the second end thereof to form first and second anchor sections.

3. The medical device anchor and delivery system of claim 1 wherein said one or more anchor sections extend arcuately outward from said anchor shaft (18) and loop back to cross and extend beyond said anchor shaft (18) to form the loop.

4. The medical device anchor and delivery system of claim 1 wherein the distal end of said tube is closed, said exit opening being formed as a side opening in said tube spaced above said closed distal end, the longitudinal movement of said anchor shaft (18) by said triggering unit (68) propelling said anchor out of said exit opening laterally of said tube.

5. The medical device anchor and delivery system of claim 4 wherein said exit opening has a lower edge spaced above the distal end of said tube, said tube having an internal curved guidewall extending from a position opposite to and above said lower edge of said exit opening to the lower edge of said opening, said curved guidewall operating to engage and guide said anchor out through said exit opening when said anchor shaft (18) moves longitudinally toward the distal end of said tube.

6. The medical device anchor and delivery system of claim 1 wherein the proximal end of said anchor shaft (18) is connected to a shaft support hub, the drive shaft (50) having a first end in engagement with said shaft support hub and operative when propelled to cause said shaft support hub to move said anchor shaft (18) longitudinally of said elongate tube to propel said expandable anchor (28) outwardly from the exit opening of said tube.

7. The medical device anchor and delivery system of claim 6 wherein said drive shaft (50) includes a second end opposite to said first end, said second end being connected to a population unit operative to propel said drive shaft (50).

8. The medical device anchor and delivery system of claim 7 wherein the proximal end of said elongate tube is connected to a tube retention sleeve, said anchor shaft (18) extending outwardly from the proximal end of said elongate tube to said shaft support hub spaced from said tube retention sleeve when said expandable anchor (28) is in said collapsed configuration within said elongate tube, said drive shaft (50) operating when propelled to move said shaft support hub toward said tube retention sleeve.

9. The medical device anchor and delivery system of claim 8 wherein said expandable anchor is formed of thermal shape memory material having a temperature transformation level where at temperatures below said temperature transformation level said shape memory material is relatively pliable and compressible and at temperatures at least at or above said temperature transformation level said shape memory material is self-expandable to a substantially rigid predetermined configuration.

10. The medical device anchor and delivery system of claim 1, wherein said tube forms one or more legs (12) of a blood clot filter for propelling one or more anchors (28) through the wall of a blood vessel from a first inner side to a second outer side, the blood clot filter having a central longitudinal axis and being collapsible to a collapsed configuration toward said longitudinal axis and expandable in an expanded configuration outwardly from said longitudinal axis for contact with said inner side of the wall of said blood vessel,
said blood clot filter including a plurality of elongate, spaced legs (12) each having a distal end and a proximal end, each of said elongate legs (12) being secured together adjacent to the longitudinal axis of said blood clot filter, said plurality of elongate spaced legs (12) being formed to extend outwardly away from said longitudinal axis to bring the distal ends thereof into contact with the first inner side of a blood vessel in the expanded configuration of said blood clot filter,
one or more of said elongate spaced legs (12) being tubular in configuration and formed by said tube containing said elongate anchor shaft (18) and expandable anchor (28), said one or more tubular elongate legs (12) each containing said expandable anchor (28) in the first collapsed condition adjacent to the distal end thereof, and
a shaft support hub connected to the first end of each elongate anchor shaft (18), said shaft support hub being spaced from the proximal ends of said elongate legs (12) when an expandable anchor (28) in the first collapsed condition is contained in said tubular elongate legs (12), said shaft support hub being movable toward said proximal ends of said elongate legs (12) to move said anchor shafts (18) longitudinally to propel said expandable anchors (28) out from the exit openings of said tubular elongate legs (12) and through the wall of a blood vessel.

11. The medical device anchor and delivery system of claim 10 wherein said expandable anchor (28) is oriented in the second expanded configuration such that the loop compresses against said blood vessel wall and changes configuration to form a smaller loop in response to forces which draw said loop against said blood vessel wall.

12. The medical device anchor and delivery system of claim 10, wherein a first drive shaft end is connected to said shaft support hub to move said shaft support hub relative to the proximal ends of said elongate legs (12).

13. The medical device anchor and delivery system of claim 12 wherein said drive shaft (50) is mounted for movement within an elongate filter centering shaft having an inner end spaced adjacent to said shaft support hub, said filter centering shaft having a plurality of elongate, spaced, centering arms secured at one end to said centering shaft inner end, said centering arms being adapted to expand outwardly into engagement with said blood vessel wall inner side.

14. The medical device anchor and delivery system of claim 10 wherein one or more of said elongate spaced legs (12) has an open proximal end, an internal passage extending from the open proximal end, a closed distal end opposite to said open proximal end and a side opening to said internal passage formed in a leg side portion above said closed distal end as said exit opening,
and a shaft propulsion unit for engaging the shaft support hub to move the elongate anchor shafts (18) longitudinally toward the closed distal ends of said one or more elongate legs (12) to propel said expandable anchors (28) out of the side openings of said one or more elongate legs (12) and through the wall of a blood vessel.

15. The medical device anchor and delivery system of claim 14 wherein said side opening has a lower edge spaced above the distal end of said respective elongate leg (12), the elongate leg (12) having an internal curved guidewall extending across said internal passage from a position opposite to and above said lower edge of the side opening to the lower edge of the side opening, said curved guidewall operating to engage and guide said anchor (28) out through said side opening when each said anchor shaft (18) moves longitudinally toward the distal end of an elongate leg.

## Patentansprüche

1. Verankerungs- und Zuführungssystem für eine medizinische Vorrichtung zum Vortreiben eines Ankers durch eine Körperwand von einer ersten Seite zu einer zweiten Seite, wobei der Anker sich gegen die zweite Seite expandiert, um fassend:
einen Ankerschaft (18), der ein erstes und ein zweites Ende aufweist,
die einander gegenüber liegen,
einen expandierbaren Anker (28) am zweiten Ende des Ankerschaftes (18), der einen oder mehrere Ankerabschnitte aufweist, wobei der expandierbare Anker (28) eine erste zusammengefaltete Konfiguration,
bei der der Anker (28) im Wesentlichen mit dem Ankerschaft (18) übereinstimmt, und eine zweite expandierte Konfiguration aufweist,
und
ein langgestrecktes Rohr mit einem offenen proximalen Ende und einem distalen Ende mit einer Ausgangsöffnung für den Anker (28), die in dem Rohr an oder benachbart, aber beabstandet zu dem distalen Ende ausgebildet ist, wobei das Rohr den Ankerschaft (18) mit dem expandierbaren Anker (28) in der zusammengefalteten Konfiguration benachbart zu der Ausgangsöffnung enthält,
**dadurch gekennzeichnet, dass**
die Verankerung der medizinischen Vorrichtung weiterhin eine Antriebsachse (50), die mit einer Auslöseeinheit (68) verbunden ist, die befestigt ist, um mit dem ersten Ende des Ankerschaftes (18) im Eingriff zu sein und den Ankerschaft (18) in Längsrichtung zu dem zweiten Ende des langgestreckten Rohrs hin zu bewegen, um den expandierbaren Anker (28) nach außen in Bezug auf die Ausgangsöffnung vorzuschieben, so dass der gesamte expandierbare Anker (28) hindurchgeht und gegen die äußere Fläche des Gefäßes expandiert, und dass in der zweiten expandierten Konfiguration des expandierbaren Ankers (28) der eine oder die mehreren Ankerabschnitte sich von dem Ankerschaft (18) nach außen in einer oder mehreren Schleifen erstrecken,
wobei jede Schleife zurückgewölbt ist, um den Ankerschaft (18) zu kreuzen.

2. Verankerungs- und Zuführungssystem für eine medizinische Vorrichtung nach Anspruch 1, bei dem der expandierbare Anker am zweiten Ende des Ankerschaftes (18) integral mit dem Ankerschaft (18) durch Teilen des Ankerschaftes (18) an seinem zweiten Ende in Längsrichtung ausgebildet ist, um den ersten und zweiten Ankerabschnitt zu bilden.

3. Verankerungs- und Zuführungssystem für eine medizinische Vorrichtung nach Anspruch 1, bei dem der eine oder die mehreren Ankerabschnitte sich bogenförmig von dem Ankerschaft (18) nach außen erstrecken und zurückschleifen, um den Ankerschaft (18) zu kreuzen und sich über den Ankerschaft (18) hinaus zu erstrecken, um die Schleife zu bilden.

4. Verankerungs- und Zuführungssystem für eine medizinische Vorrichtung nach Anspruch 1, bei dem das distale Ende des Rohrs geschlossen ist, wobei die Ausgangsöffnung als eine Seitenöffnung in dem Rohr ausgebildet ist, die beabstandet zu dem geschlossenen distalen Ende ist, wobei die Längsbewegung des Ankerschaftes (18) durch die Auslöseeinheit (68) den Anker aus der Ausgangsöffnung seitlich des Rohres herausdrückt.

5. Verankerungs- und Zuführungssystem für eine medizinische Vorrichtung nach Anspruch 4, bei dem die Ausgangsöffnung eine mit Abstand über dem distalen Ende des Rohres liegende untere Kante aufweist, wobei das Rohr eine innere gekrümmte Führungswand hat, die sich von einer Position entgegengesetzt zu und über der unteren Kante der Ausgangsöffnung zu der unteren Kante der Öffnung erstreckt, wobei die gekrümmte Führungswand ausgeführt ist, um mit dem Anker in Kontakt zu treten und ihn nach außen durch die Ausgangsöffnung zu führen, wenn der Ankerschaft (18) sich längs zu dem distalen Ende des Rohrs bewegt.

6. Verankerungs- und Zuführungssystem für eine medizinische Vorrichtung nach Anspruch 1, bei dem das proximale Ende des Ankerschaftes (18) mit einer Schaftstütznabe verbunden ist, wobei die Antriebsachse (50) ein erstes Ende im Eingriff mit der Schaftstütznabe aufweist und operativ ist, wenn sie angetrieben wird, um zu bewirken, dass die Ankerstütznabe den Ankerschaft (18) in Längsrichtung des langgestreckten Rohrs bewegt, um den expandierbaren Anker (28) nach außen aus der Ausgangsöffnung des Rohrs vorzuschieben.

7. Verankerungs- und Zuführungssystem für eine medizinische Vorrichtung nach Anspruch 6, bei dem die Antriebsachse (50) ein zweites Ende, entgegengesetzt zu dem ersten Ende einschließt, wobei das zweite Ende mit einer Antriebseinheit verbunden ist, die ausgebildet ist, um die Antriebsachse (50) vorzutreiben.

8. Verankerungs- und Zuführungssystem für eine medizinische Vorrichtung nach Anspruch 7, bei dem das proximale Ende des langgestreckten Rohres mit einer Rohrrückhaltehülse verbunden ist, wobei der Ankerschaft (18) sich von dem proximalen Ende des langgestreckten Rohrs nach außen zu der Schaftstütznabe erstreckt, die zu der Rohrrückhaltehülse beabstandet ist, wenn der expandierbare Anker (28) in seiner zusammengefalteten Konfiguration in dem langgestreckten Rohr ist, wobei die Antriebsachse (50), wenn sie vorgetrieben wird, operativ ist, um die Schaftstütznabe zu der Rohrrückhaltehülse zu bewegen.

9. Verankerungs- und Zuführungssystem für eine medizinische Vorrichtung nach Anspruch 8, bei dem der expandierbare Anker aus thermischem Formspeichermaterial geformt ist, das ein Temperaturtransformationsniveau aufweist, bei dem bei Temperaturen unter dem Temperaturtransformationsniveau das Formspeichermaterial relativ biegsam und komprimierbar ist, und bei Temperaturen von mindestens oder über dem Temperaturtransformationsniveau das Formspeichermaterial selbstexpandierbar in eine im Wesentlichen starre vorbestimmte Konfiguration ist.

10. Verankerungs- und Zuführungssystem für eine medizinische Vorrichtung nach Anspruch 1, bei dem das Rohr einen oder mehrere Arme (12) eines Blutgerinnselfilters zum Vortreiben eines oder mehrerer Anker (28) durch die Wand eines Blutgefäßes von einer ersten inneren Seite zu einer zweiten äußeren Seite bildet, wobei das Blutgerinnselfilter eine zentrale Längsachse aufweist und zusammenfaltbar zu einer zusammengefalteten Konfiguration zu der Längsachse hin und expandierbar in eine expandierte Konfiguration von der Längsachse nach außen für den Kontakt mit der Innenseite der Wand des Blutgefäßes ist, wobei das Blutgerinnselfilter eine Mehrzahl von langgestreckten beabstandeten Armen (12) einschließt, von denen jeder ein distales Ende und ein proximales Ende aufweist und die langgestreckten Arme (12) miteinander benachbart zu der Längsachse des Blutgerinnselfilters befestigt sind, wobei die Mehrzahl von langgestreckten beabstandeten Armen (12) ausgebildet ist, um sich von der Längsachse nach außen weg zu erstrecken, um ihre distalen Enden in Kontakt mit der ersten Innenseite eines Blutgefäßes in der expandierten Konfiguration des Blutgerinnselfilters zu bringen,
wobei einer oder mehrere der langgestreckten beabstandeten Arme (12) in seiner Konfiguration rohrförmig ist und von dem Rohr, das den langgestreckten Ankerschaft (18) und den expandierbaren Anker (28) enthält, gebildet wird, wobei der eine oder die mehreren rohrförmigen langgestreckten Arme (12) jeder den expandierbaren Anker (28) in dem ersten zusammengefalteten Zustand benachbart zu seinem distalen Ende enthält, und
eine Schaftstütznabe, die mit dem ersten Ende jedes langgestreckten Ankerschaftes (18) verbunden ist, wobei die Schaftstütznabe zu den proximalen Enden der langgestreckten Arme (12) beabstandet ist, wenn ein expandierbarer Anker (28) in dem ersten zusammengefalteten Zustand in den rohrförmigen langgestreckten Armen (12) enthalten ist, wobei die Schaftstütznabe zu den proximalen Enden der langgestreckten Arme (12) bewegbar ist, um die Ankerschäfte (18) in Längsrichtung zu bewegen, um die expandierbaren Anker (28) aus den Ausgangsöffnungen der rohrförmigen langgestreckten Arme (12) heraus und durch die Wand des Blutgefäßes vorzuschieben.

11. Verankerungs- und Zuführungssystem für eine medizinische Vorrichtung nach Anspruch 10, bei dem der expandierbare Anker (28) in der zweiten expandierten Konfiguration so ausgerichtet ist, dass die Schleife gegen die Blutgefäßwand drückt und die Konfiguration ändert, um eine kleinere Schleife in Antwort auf Kräfte zu bilden, die die Schleife gegen die Blutgefäßwand ziehen.

12. Verankerungs- und Zuführungssystem für eine medizinische Vorrichtung nach Anspruch 10, bei dem ein erstes Ende der Antriebsachse mit der Schaftstütznabe verbunden ist, um die Schaftstütznabe relativ zu den proximalen Enden der langgestreckten Arme (12) zu bewegen.

13. Verankerungs- und Zuführungssystem für eine medizinische Vorrichtung nach Anspruch 12, bei dem die Antriebsachse (50) zur Bewegung in einem langgestreckten Filterzentrierschaft befestigt ist, der ein inneres Ende aufweist, das zu der Schaftstütznabe beabstandet ist, wobei der Filterzentrierschaft eine Vielzahl von langgestreckten, beabstandeten Zentrierarmen aufweist, die an einem Ende mit dem inneren Ende des Zentrierschaftes verbunden sind, wobei die Zentrierarme ausgebildet sind, um nach außen in Eingriff mit der Innenseite der Blutgefäßwand zu expandieren.

14. Verankerungs- und Zuführungssystem für eine medizinische Vorrichtung nach Anspruch 10, bei dem einer oder mehrere der langgestreckten beabstandeten Arme (12) ein offenes proximales Ende, einen sich von dem offenen proximalen Ende erstreckenden inneren Durchgang, ein geschlossenes distales Ende, gegenüberliegend zu dem offenen proximalen Ende und eine Seitenöffnung zu dem inneren Durchgang, die in einem Seitenbereich des Arms über dem geschlossenen distalen Ende ausgebildet ist, als die Ausgangsöffnung aufweist,
und eine Schaftvortriebseinheit für den Eingriff mit der Schaftstütznabe vorgesehen ist, um die langgestreckten Ankerschäfte (18) in Längsrichtung zu den geschlossenen distalen Enden eines oder mehrerer langgestreckter Arme (12) hin zu bewegen, um die expandierbaren Anker (28) aus den Seitenöffnungen des einen oder der mehreren langgestreckten Arme (12) und durch die Wand eines Blutgefäßes vorzuschieben.

15. Verankerungs- und Zuführungssystem für eine medizinische Vorrichtung nach Anspruch 14, bei dem die Seitenöffnung eine untere, zu dem distalen Ende des jeweiligen langgestreckten Arms (12) beabstandete Kante aufweist, wobei der langgestreckte Arm (12) eine innere gekrümmte Führungswand hat, die sich durch den inneren Durchgang von einer Position entgegengesetzt zu und über der unteren Kante der Seitenöffnung zu der unteren Kante der Seitenöffnung erstreckt, wobei die gekrümmte Führungswand operativ ist, um den Anker (28) zu kontaktieren und nach außen durch die Seitenöffnung zu führen, wenn jeder Ankerschaft (18) sich längs zu dem distalen Ende eines langgestreckten Arms bewegt.

## Revendications

1. Système d'ancrage et de déploiement d'un dispositif médical pour propulser une ancre à travers une paroi corporelle depuis un premier côté vers un second côté où ladite ancre se déploie contre ledit second côté, comprenant :
un arbre d'ancrage (18) ayant une première et une seconde extrémités opposées,
une ancre extensible (28) à la seconde extrémité dudit arbre d'ancrage (18) ayant une ou plusieurs sections d'ancrage, ladite ancre extensible (28) ayant une première configuration affaissée, dans laquelle ladite ancre (28) est sensiblement co-extensible avec ledit arbre d'ancrage (18) et une seconde configuration déployée, et
un tube allongé ayant une extrémité proximale ouverte et une extrémité distale avec une ouverture de sortie pour ladite ancre (28) étant formée dans ledit tube au niveau ou près de ladite extrémité distale, mais espacée de celle-ci, ledit tube contenant ledit arbre d'ancrage (18) avec ladite ancre extensible (28), dans ladite configuration affaissée, adjacente à ladite ouverture de sortie,
**caractérisé en ce que**
ledit dispositif médical comprend en outre un arbre de commande (50) connecté à une unité de déclenchement (68) montée de façon à mettre en prise ladite première extrémité dudit arbre d'ancrage (18) et à déplacer ledit arbre d'ancrage (18) longitudinalement vers la seconde extrémité dudit tube allongé afin de propulser ladite ancre extensible (28) vers l'extérieur, depuis ladite ouverture de sortie, de sorte que l'ensemble de l'ancre extensible (28) passe à travers et se déploie contre la surface extérieure du vaisseau et **en ce que**
dans la seconde configuration déployée de ladite ancre extensible (28), ladite une ou
plusieurs sections d'ancrage s'étendent vers l'extérieur depuis ledit arbre d'ancrage (18) en une ou plusieurs boucles, chaque boucle s'incurvant vers l'arrière pour croiser l'arbre d'ancrage (18).

2. Système d'ancrage et de déploiement d'un dispositif médical selon la revendication 1, dans lequel l'ancre extensible, à la seconde extrémité dudit arbre d'ancrage (18), est intégralement formée d'un arbre d'ancrage (18) en séparant ledit arbre d'ancrage (18) longitudinalement à sa seconde extrémité pour former une première et une seconde sections d'ancrage.

3. Système d'ancrage et de déploiement d'un dispositif médical selon la revendication 1, dans lequel une ou plusieurs sections d'ancrage s'étendent de manière arquée vers l'extérieur dudit arbre d'ancrage (18) et font une boucle en arrière pour croiser et s'étendre au-delà dudit arbre d'ancrage (18) afin de former la boucle.

4. Système d'ancrage et de déploiement d'un dispositif médical selon la revendication 1, dans lequel l'extrémité distale dudit tube est fermée, ladite ouverture de sortie étant formée comme une ouverture latérale dans ledit tube, espacée au-dessus de ladite extrémité distale fermée, le mouvement longitudinal dudit arbre d'ancrage (18) par ladite unité d'enclenchement (68) propulsant ladite ancre hors de ladite ouverture de sortie latéralement par rapport audit tube.

5. Système d'ancrage et de déploiement d'un dispositif médical selon la revendication 4, dans lequel ladite ouverture de sortie a un bord inférieur espacé au-dessus de l'extrémité distale dudit tube, ledit tube ayant une paroi de guidage incurvée interne, s'étendant depuis une position opposée et supérieure audit bord inférieur de ladite ouverture de sortie vers le bord inférieur de ladite ouverture, ladite paroi de guidage incurvée fonctionnant de façon à mettre en prise et à guider ladite ancre hors de ladite ouverture de sortie, quand ledit arbre d'ancrage (18) se déplace longitudinalement vers l'extrémité distale dudit tube.

6. Système d'ancrage et de déploiement d'un dispositif médical selon la revendication 1, dans lequel l'extrémité proximale dudit arbre d'ancrage (18) est raccordée à un moyeu de support de l'arbre, l'arbre de commande (50) ayant une première extrémité en prise avec ledit moyeu de support d'arbre et opérationnelle lorsqu'il est propulsé pour provoquer le déplacement par ledit moyeu de support d'arbre dudit arbre d'ancrage (18) longitudinalement par rapport audit tube allongé, afin de propulser ladite ancre extensible (28) vers l'extérieur, depuis l'ouverture de sortie dudit tube.

7. Système d'ancrage et de déploiement d'un dispositif médical selon la revendication 6, dans lequel ledit arbre de commande (50) comprend une seconde extrémité opposée à ladite première extrémité, ladite seconde extrémité étant raccordée à une unité de propulsion opérationnelle pour propulser ledit arbre de commande (50).

8. Système d'ancrage et de déploiement d'un dispositif médical selon la revendication 7, dans lequel l'extrémité proximale dudit tube allongé est raccordée à un manchon de retenue de tube, ledit arbre d'ancrage (18) s'étendant vers l'extérieur depuis l'extrémité proximale dudit tube allongé audit moyeu de support d'arbre, espacé dudit manchon de retenue de tube quand ladite ancre extensible (28) est dans ladite configuration affaissée dans ledit tube allongé, ledit arbre de commande (50) fonctionnant quand il est propulsé pour déplacer ledit moyeu de support d'arbre vers ledit manchon de retenue de tube.

9. Système d'ancrage et de déploiement d'un dispositif médical selon la revendication 8, dans lequel ladite ancre extensible est formée d'un matériau à mémoire de forme thermique, ayant un niveau de transformation à la température où, à des températures inférieures audit niveau de transformation à la température, ledit matériau à mémoire de forme est relativement pliable et compressible, et à des températures au moins égales ou supérieures audit niveau de transformation de température, ledit matériau à mémoire de forme est auto-dilatable en une configuration prédéterminée sensiblement rigide.

10. Système d'ancrage et de déploiement d'un dispositif médical selon la revendication 1, dans lequel ledit tube forme une ou plusieurs pattes (12) d'un filtre à caillot sanguin pour propulser une ou plusieurs ancres (28) à travers la paroi d'un vaisseau sanguin, depuis un premier côté interne vers un second côté externe, ledit filtre à caillot sanguin ayant un axe longitudinal central et pouvant s'affaisser dans une configuration affaissée, vers ledit axe longitudinal et extensible dans une configuration déployée vers l'extérieur depuis ledit axe longitudinal pour entrer en contact avec ledit côté intérieur de la paroi dudit vaisseau sanguin,
ledit filtre à caillot sanguin comprenant une pluralité de pattes allongées, espacées (12), ayant chacune une extrémité distale et une extrémité proximale, chacune desdites pattes allongées (12) étant fixée ensemble de manière adjacente à l'axe longitudinal dudit filtre à caillot sanguin, ladite pluralité de pattes espacées allongées (12) étant formée pour s'étendre vers l'extérieur, éloignée dudit axe longitudinal, afin de mettre les extrémités distales en contact avec le premier côté interne d'un vaisseau sanguin dans la configuration dilatée dudit filtre à caillot sanguin,
une ou plusieurs pattes espacées allongées (12) étant de configuration tubulaire et formées par ledit tube contenant ledit arbre d'ancrage allongé (18) et l'ancre extensible (28), lesdites une ou plusieurs pattes allongées tubulaires (12) contenant chacune ladite ancre extensible (28) dans une première condition affaissée adjacente à son extrémité distale, et
un moyeu de support d'arbre raccordé à la première extrémité de chaque arbre d'ancrage allongé (18), ledit moyeu de support d'arbre étant espacé des extrémités proximales desdites pattes allongées (12) quand une ancre extensible (28) dans la première condition affaissée est contenue dans lesdites pattes allongées tubulaires (12), ledit moyeu de support d'arbre étant mobile vers lesdites extrémités proximales desdites pattes allongées (12) afin de déplacer lesdits arbres d'ancrage (18) longitudinalement pour propulser lesdites ancres extensibles (28) hors des ouvertures de sortie desdites pattes allongées tubulaires (12) et à travers la paroi d'un vaisseau sanguin.

11. Système d'ancrage et de déploiement d'un dispositif médical selon la revendication 10, dans lequel ladite ancre extensible (28) est orientée dans la seconde configuration étendue, de sorte que la boucle comprime ladite paroi du vaisseau sanguin et change la configuration pour former une boucle plus petite en réponse à des forces qui attirent ladite boucle contre ladite paroi de vaisseau sanguin.

12. Système d'ancrage et de déploiement d'un dispositif médical selon la revendication 10, dans lequel une première extrémité d'arbre de commande est raccordée audit moyeu de support d'arbre pour déplacer ledit moyeu de support d'arbre relativement aux extrémités proximales desdites pattes allongées (12).

13. Système d'ancrage etde déploiement d'un dispositif médical selon la revendication 12, dans lequel ledit arbre de commande (50) est monté de façon à permettre le mouvement dans un arbre de centrage de filtre allongé, ayant une extrémité interne espacée adjacente audit moyeu de support d'arbre, ledit arbre de centrage de filtre ayant une pluralité de bras de centrage allongés, espacés, fixés à une extrémité vers ladite extrémité interne d'arbre de centrage, lesdits bras de centrage étant adaptés pour s'étendre vers l'extérieur en prise avec ledit côté interne de la paroi de vaisseau sanguin.

14. Système d'ancrage et de déploiement d'un dispositif médical selon la revendication 10, dans lequel une ou plusieurs desdites pattes allongées espacées (12) a une extrémité proximale ouverte, un passage interne s'étendant depuis l'extrémité proximale ouverte, une extrémité distale fermée opposée à ladite extrémité proximale ouverte et une ouverture latérale audit passage interne formée dans une partie latérale de patte au-dessus de ladite extrémité distale fermée comme ladite ouverture de sortie,
et une unité de propulsion d'arbre pour mettre en prise le moyeu de support d'arbre pour déplacer les arbres d'ancrage allongés (18) longitudinalement vers les extrémités distales fermées desdites une ou plusieurs pattes allongées (12), afin de propulser lesdites ancres extensibles (28) hors des ouvertures latérales desdites une ou plusieurs pattes allongées (12) et à travers la paroi d'un vaisseau sanguin.

15. Système d'ancrage et de déploiement d'un dispositif médical selon la revendication 14, dans lequel ladite ouverture latérale a un bord inférieur espacé au-dessus de l'extrémité distale de ladite patte allongée respective (12), la patte allongée (12) ayant une paroi de guidage incurvée interne s'étendant à travers ledit passage interne, depuis une position opposée et supérieure audit bord inférieur de l'ouverture latérale vers le bord inférieur de l'ouverture latérale, ladite paroi de guidage incurvée fonctionnant de façon à mettre en prise et à guider ladite ancre (28) en dehors, à travers ladite ouverture latérale quand chacun desdits arbres d'ancrage (18) se déplace longitudinalement vers l'extrémité distale d'une patte allongée.
